# EUROPEAN PATENT APPLICATION

(11) **EP 4 317 411 A1**
(43) Date of publication of application: **07.02.2024**
(21) Application number: 22774314.3
(22) Date of filing: 24.03.2022
(51) Int. Cl.: C12N 1/20, C12P 21/00, C12P 19/04, C12P 13/04, A61K 8/99, A61P 17/00, A61P 31/10, A61P 37/04, A61Q 17/00, A61Q 19/00, C12R 1/225

(54) **LACTOBACILLUS RHAMNOSUS, FERMENT LYSATE FOR REGULATING SKIN MICROECOLOGY, PREPARATION METHOD, AND APPLICATION**

(30) Priority: 24.03.2021 CN 202110316031
(71) Applicant: Bloomage Biotechnology Corporation Limited, Jinan, Shandong 250101 (CN)
(72) Inventor: LU, Zhen, Jinan, Shandong 250101 (CN); WEI, Yujie, Jinan, Shandong 250101 (CN); SUN, Yuanjun, Jinan, Shandong 250101 (CN); DU, Ranran, Jinan, Shandong 250101 (CN); ZHAO, Yan, Jinan, Shandong 250101 (CN); GUO, Xueping, Jinan, Shandong 250101 (CN)
(74) Representative: Abel & Imray LLP
(86) International application number: PCT/CN2022/082787
(87) International publication number: WO 2022/199660

(57) **Abstract**

Disclosed are a *Lactobacillus rhamnosus* strain having accession number CCTCC NO: M 2021185, a ferment lysate prepared thereby for regulating skin microecology, and a preparation method for the ferment lysate. The ferment lysate comprises the following components in mass percentages of same in the ferment lysate: 0.2-1% of proteins, 0.3-0.8% of polysaccharides, and 0.2-1% of amino acids. The ferment lysate obtained by using the above bacterial strain for fermentation can strongly inhibit the reproduction and growth of pathogenic bacteria, has a promotion effect on some of probiotics, has a strong inhibition effect on pathogenic hyphae of *Candida albicans,* can not only generate antimicrobial peptides during fermentation, but also increase the expression level of antimicrobial peptides in skin cells at the genetic level and the protein level, thereby improving skin immunity.

## Description

### TECHNICAL FIELD

The application relates to the technical field of cosmetic raw materials, in particular to a *Lactobacillus rhamnosus* strain, a ferment lysate for regulating skin microecology, a preparation method and an application thereof.

### BACKGROUND ART

Skin microecology is to study the relationship between the structure and function of human skin microbiota and human skin itself, so as to explain the interaction relationship between skin and microorganisms, and the nature of abnormal changes in skin. The skin microecology is an ecological system mainly composed of various microorganisms such as bacteria, fungi, viruses, mites, and arthropods, as well as tissues, cells, various secretions, and microenvironments on the skin surface. Skin micro-ecological balance is formed by the interaction of skin microbes, host and external environment. Human skin microecology is directly related to human health. When the human body microecology is abnormal, symptoms such as burning, tingling, itching, and tightness of sensitive skin will appear.

The skin barrier is composed of the following four layers: the outermost microbial barrier, chemical barrier, physical barrier, and immune barrier. Particularly, the four types of barrier functions are balanced with each other. Under normal conditions, they have the function of quickly repairing the barrier and immune homeostasis. An imbalance at a certain layer causes the skin's susceptibility to external pressure and skin diseases. Skin immunity and skin barrier are closely related, microbial barrier and immune barrier together constitute the skin immune function.

The low immune function of the skin is often manifested first in the stratum corneum barrier, and the micro-ecological barrier on it (i.e., the microbiota that is symbiotic with the skin) is particularly easy to be ignored. The basic characteristics of human skin are dry, cool and weakly acidic. These conditions are not an ideal environment for microorganisms to survive. However, due to the distribution of large number of folds, hair follicles and sweat glands on the skin, various types of microorganisms including bacteria, fungi, and parasites have colonized on the skin during the evolution process. It is predicted that each square centimeter of skin may be colonized with up to billions of microorganisms.

Through the analysis of the microecology of the human epidermis, skin care products can increase the diversity of skin beneficial bacteria by adding certain ingredients, or inhibit the invasion and proliferation of pathogenic bacteria by enhancing the reproduction of probiotics. This concept can be modeled after the intestinal probiotics of the human body, eating foods containing probiotics may help regulate intestinal health, thus the overall status of human immune function can be improved by regulation of intestinal microbiota. Based on this theory, active probiotics are screened, and fermentation products containing probiotics are added to various skin care products artificially. Particularly, the active protein produced by fermentation of probiotics can be used as an antimicrobial peptide to inhibit the growth of the harmful transient bacteria (i.e., microorganisms growing on the human body due to unconscious contact between human and the external environment caused by a series of behaviors during social and natural activities). At the same time, the active ingredients released by cleavage of probiotics provide nutrition to human skin. Therefore, how to extract all the active substances obtained from the fermentation process of probiotics without destruction in a gentle and complete manner is particularly important. In cosmetic regulations, live bacteria cannot be directly added to products as raw materials. A large number of studies have shown that, the dead bacteria of lactic acid bacteria can also exhibit some functions of the live bacteria preparation, and there is a certain dose correlation. Therefore, the inactivated lactic acid bacteria preparation provides another relatively stable direction for the industrialization of microecological preparations. In the existing products, the product is often obtained by violent crushing of the bacterial cells such as high temperature heating, strong acid and strong alkali, etc., although efficient crushing of the bacterial cells can be achieved by such methods, the active ingredients will be affected by the impact of the crushing process, and be seriously destroyed to be inactive; or the bacteria are inactivated in a mild way, while the entire structure of the bacteria remains relatively intact, so that the active ingredients in the relatively complete cell structure in cosmetics cannot be efficiently absorbed and utilized by human skin cells to exert its efficacy. Actually, the highly active substances are the ingredients such as oligosaccharides, oligopeptides, amino acids, etc. that make up the entire structure of cells. Only small molecular substances can be absorbed and utilized by skin cells. Therefore, a method for gently degrading the cell structure of probiotics is needed to make the resulting substance to serve as prebiotics to work very well.

In addition, the selection of probiotics is also very important. Different lactic acid bacteria have different effects. For example, *Bifidobacterium* has a repairing and protective function, *Lactobacillus plantarum* usually has an antioxidant effect, and *Lactobacillus rhamnosus* has an effect different from these types of lactic acid bacteria, it is often applied to food products as a safe strain, and can improve human immunity after use. The source of the strain screened in this application is obtained from breast milk and has a large amount of antibodies, such a *Lactobacillus rhamnosus* strain colonized for a long time in this environment has experienced long-term domestication during its own growth process, and has a higher function of metabolizing immune active substances. The screened strain is prepared according to the process of the application, and its fermentation products have been found to express antimicrobial peptides at a high level through efficacy experiments, also have the ability to regulate the diversity of skin bacterial colonies, and effectively inhibit the transformation process of pathogenicity state of *Candida albicans.*
(1) A preparation method of inactivated lactic acid bacteria preparation is disclosed in Chinese invention application CN200810068138.X, titled " Microecological Preparation of Inactivated Lactic Acid Bacteria and Preparation Method Thereof", wherein the inactivation methods include heat inactivation, freezing inactivation and formaldehyde inactivation etc., the chemical inactivation method stated in the application adopts fungicides such as formaldehyde to inactivate, and the safety of the product is not guaranteed.
(2) Heat inactivation is used in Chinese invention application CN201180004722.9 of Cell Biotech Co. Ltd. from Korea, and heat inactivation process is also used in Chinese invention application CN200910206343.2 of House Wellness Food Corp. from Japan.
(3) An inactivated lactic acid bacterium liquid preparation and preparing method thereof is disclosed in Chinese invention application CN200810068138.X, titled "Inactivated Lactic Acid Bacterium Liquid Preparation and Preparing Method Thereof". It provides a liquid preparation of inactivated lactic acid bacteria, wherein the acidic additives such as betaine hydrochloride, glucosamine hydrochloride, γ-aminobutyric acid are selected as inactivators. Hydrochloride substances are added in the entire inactivation process, and the bacterial cells are inactivated mainly relying on low pH, and various active substances may also be inactivated at low pH, especially some basic amino acids such as lysine, arginine and histidine etc. are easily inactivated under strong acid. In addition, since lactic acid bacteria are suitable for a low pH environment for a long time, although they can be inactivated at low pH, the structure of lactic acid bacteria cells will not be greatly damaged. In this way, raw materials which are inactivated but maintain a complete cell structure are used in cosmetics, but the functional ingredient in the form of cells cannot be absorbed and utilized by skin cells, and will not exert the inherent efficacy of lactic acid bacteria.
(4) A *Lactobacillus Rhamnosus* strain and use thereof is disclosed in Chinese invention application CN110122877A, titled *"Lactobacillus Rhamnosus* and Use Thereof", and use of *Lactobacillus rhamnosus* in the regulation of immunity is also described. This *Lactobacillus rhamnosus* strain was preserved in the General Microbiology Center of the China Microbiological Culture Collection Management Committee on August 10, 2017, with an accession number CGMCC No.14511 and a classification name: *Lactobacillus rhamnosus.* The *Lactobacillus rhamnosus* strain has a good immune regulation effect on the body; and its inhibitory effect on the expression of inflammatory factors, the promotion effect on the expression of anti-inflammatory factors, and the promotion effect on the proliferation of lymphocytes are significantly better than the existing commercial *Lactobacillus rhamnosus* strain, thus its immune regulation function on the body is significantly better than the existing *Lactobacillus rhamnosus* strains. In the course of the experiment, the live bacterial cells were used to test the efficacy of the bacteria, and the extracellular products of the bacterial cells were discarded; additionally, the active substances in the cells could not be fully released without the wall-breaking process, and the activity was greatly reduced. As a result, this strain only has the activity of general *Lactobacillus rhamnosus,* for example, the expression of antimicrobial peptide gene and the protein level, the regulation of pathogenic bacteria such as *Candida albicans,* and the analysis and comparison of active substances are all insufficient.

### SUMMARY OF THE APPLICATION

In view of the above problems, the present application provides a bacterial strain and a ferment lysate obtained by fermentation with the bacterial strain. The ferment lysate product is stable, has a high content of active substances, and is safe and healthy to meet the requirements of cosmetic raw materials.

The bacterial strain described in the present application is *Lactobacillus rhamnosus* 11-7, which is isolated from newborn breast milk. The bacterial strain is preserved in the China Center for Type Culture Collection, with accession number CCTCC NO: M 2021185.

The above bacterial strain is used to carry out fermentation and culture in the present application, and then a ferment lysate is obtained by enzymolysis; the ferment lysate can inhibit the reproduction and growth of pathogenic bacteria, promote some probiotics, and have a strong inhibitory effect on the pathogenic hyphae of *Candida albicans.* Additionally, the bacterial strain itself can not only generate antimicrobial peptides during fermentation, but also up-regulate the expression level of antimicrobial peptides in skin cells at the genetic level and the protein level, thereby improving skin immunity, and as a cosmetic raw material it can significantly regulate the skin micro-ecological environment.

The specific technical solutions of the present application are as follows:
1. A *Lactobacillus rhamnosus* 11-7 strain, which is preserved in the China Center for Type Culture Collection (CCTCC) with accession number CCTCC NO: M 2021185.
2. Use of *Lactobacillus rhamnosus* 11-7 strain with accession number CCTCC NO: M 2021185 in the field of fermentation, preferably in the field of producing ferment lysates by fermentation.
3. A ferment lysate, which comprises proteins, polysaccharides and amino acids, wherein based on the mass percentage in the ferment lysate, the content of the proteins is 0.2-1%, preferably 0.6-0.9%; the content of the polysaccharides is 0.3-0.8%, preferably 0.4-0.6%; and the content of the amino acids is 0.2-1%, preferably 0.4-0.5%.
4. The ferment lysate according to item 3, wherein the ferment lysate is obtained by performing fermentation and enzymolysis by using *Lactobacillus rhamnosus* strain with accession number CCTCC NO: M 2021185,
   preferably, the ferment lysate is prepared by a method comprising the steps of:
   inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells; preferably, the fermentation medium contains carbon source, nitrogen source and inorganic salt; and preferably, the addition amount of the carbon source is 1-5% (w/v), preferably 1-4% (w/v), the addition amount of the nitrogen source is 0.5-2wt%, and the addition amount of the inorganic salt is 0.1-1wt%; and
   performing enzymolysis of the fermentation bacterial cells to obtain a ferment lysate.
5. The ferment lysate according to item 4, wherein the enzymes used in the enzymolysis are lysozyme, neutral protease and snailase; preferably, the specific enzyme activity of the lysozyme is 2000-20000IU/mg, and the specific enzyme activity of the neutral protease is 50000-100000 IU/g.
6. The ferment lysate according to item 4 or 5, wherein the time period of the enzymolysis is 1-2h, and the temperature of the enzymolysis is 35-40°C.
7. The ferment lysate according to any one of items 5-6, wherein for 1 g of the fermentation bacterial cells, the addition amount of lysozyme is 0.01-0.1 mg, the addition amount of snailase is 1-10 mg, and the addition amount of neutral protease is 0.01-0.1mg.
8. The ferment lysate according to any one of items 4-7, wherein the supernatant obtained after centrifugation is filtered through a filter membrane to obtain a second fermentation broth.
9. The ferment lysate according to item 8, wherein after enzymolysis, before obtaining the ferment lysate, the method for preparing a ferment lysate further comprises the following steps:
   centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.
10. The ferment lysate according to item 9, wherein the concentration of the sodium chloride is 3-5wt%.
11. The ferment lysate according to item 9 or 10, wherein the mass-volume percentage of the cell debris in the second fermentation broth is 1-10%, preferably 5-8%.
12. A method for preparing a ferment lysate, comprising the step of:
   preparing a ferment lysate by using *Lactobacillus rhamnosus* 11-7 strain with accession number CCTCC NO: M 2021185.
13. The method according to item 12, wherein the ferment lysate is obtained by performing fermentation and enzymolysis by using the bacterial strain.
14. The method according to item 13, wherein the method comprises the steps of:
   inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells; preferably, the fermentation medium contains carbon source, nitrogen source and inorganic salt; and preferably, the addition amount of the carbon source is 1-5% (w/v), preferably 1-4% (w/v), the addition amount of the nitrogen source is 0.5-2wt%, and the addition amount of the inorganic salt is 0.1-1wt%; and
   performing enzymolysis of the fermentation bacterial cells to obtain a ferment lysate.
15. The method according to item 13 or 14, wherein the enzymes used in the enzymolysis are lysozyme, neutral protease and snailase; preferably, the specific enzyme activity of the lysozyme is 2000-20000IU/mg, and the specific enzyme activity of the neutral protease is 50000-100000 IU/g.
16. The method according to item 14 or 15, wherein the time period of the enzymolysis is 1-2h, and the temperature of the enzymolysis is 35-40°C.
17. The method according to item 15 or 16, wherein for 1 g of the fermentation bacterial cells, the addition amount of lysozyme is 0.01-0.1 mg, the addition amount of snailase is 1-10 mg, and the addition amount of neutral protease is 0.01-0.1mg.
18. The method according to any one of items 14-17, wherein the supernatant obtained after centrifugation is filtered through a filter membrane to obtain a second fermentation broth.
19. The method according to item 18, wherein after enzymolysis, before obtaining the ferment lysate, the method further comprises the following steps:
   centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.
20. The method according to item 19, wherein the concentration of the sodium chloride is 3-5 wt%.
21. The method according to item 19 or 20, wherein the mass-volume percentage of the cell debris in the second fermentation broth is 1-10%, preferably 5-8%.
22. Use of the ferment lysate according to any one of items 3-11 or the ferment lysate prepared by the method according to any one of items 12-21 in the field of cosmetics.
23. The use according to item 22, wherein the ferment lysate is used for regulating skin microecology.

### The Effect of the Application

The fermented strain selected in the present application is probiotics, and a strain of *Lactobacillus rhamnosus* is isolated and screened from breast milk. After fermentation, the resulting fermentation broth is subjected to a process of crushing the bacterial cells; a mild way of crushing the bacterial cells by enzymolysis in combination with hypertonic solution is explored to inactivate and crush the bacterial cells. The bacterial cells are crushed to obtain a variety of active ingredients as small molecules that are easily absorbed, such as amino acids, polysaccharides, polypeptides, etc., so that the materials are transformed from cell morphology that cannot be absorbed by the human skin to a small molecule state that can be easily absorbed. Moreover, by treating the bacterial cells and the fermentation supernatant separately, it is possible to reduce the amount of enzymolysis solution to be added, reduce the risk of skin allergies, and also preserve the extracellular active ingredients present in the supernatant. The whole process not only maintains the types and activities of functional substances in the whole fermentation, but also converts them into forms that are easy to absorb. The production cycle is short, and the process is controllable and easy to scale up. The product is stable with high content of active substances which is easy to absorb, thereby having good efficacy. The product is also safe and healthy, thereby conforming to the characteristics of cosmetic raw materials. At the same time, the ferment lysate prepared by this bacterial strain with the process of the present application has the following characteristics:
1. It strongly inhibits the reproduction and growth of pathogenic bacteria, and has a promoting effect on some probiotics, especially having strong inhibition effect on the pathogenic hyphae of *Candida albicans.*
2. During the fermentation process, it can not only produce antimicrobial peptides, but also up-regulate the expression of antimicrobial peptides in skin cells at the gene level and protein level, thereby improving skin immunity; as a cosmetic raw material it can significantly regulate the skin micro-ecological environment.

### BRIEF DESCRIPTION OF THE DRAWINGS

Fig. 1 is a schematic diagram showing the electrophoresis result in Example 1;

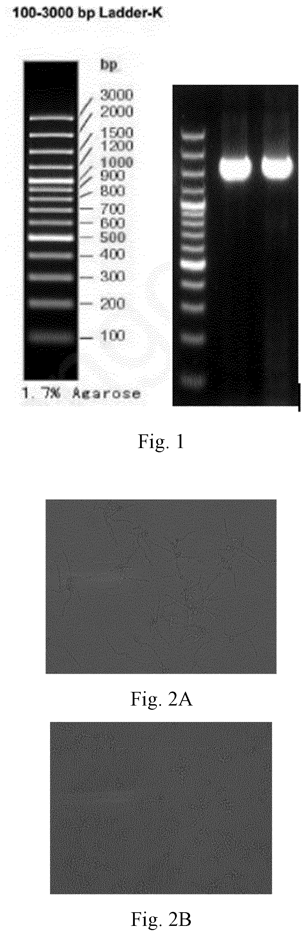

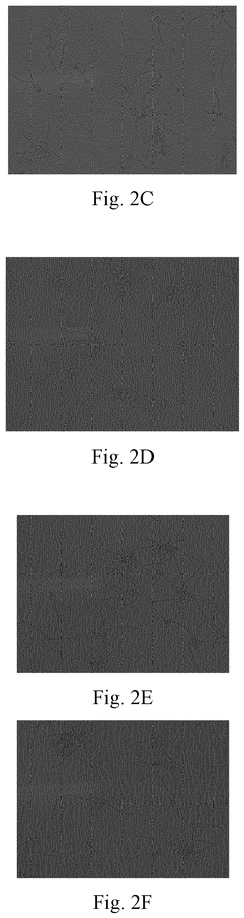

Fig. 2 is a schematic diagram showing the growth of *Candida albicans* hyphae in Test Example 5, wherein Fig. 2A is a schematic diagram showing the growth of *Candida albicans* hyphae in GE medium, Fig. 2B is a schematic diagram showing the growth of *Candida albicans* hyphae in GE medium with addition of 5% S1, Fig. 2C is a schematic diagram showing the growth of *Candida albicans* hyphae with addition of 5% C1, Fig. 2D is a schematic diagram showing the growth of *Candida albicans* hyphae in GE medium with addition of 5% C2, Fig. 2E is a schematic diagram showing the growth of *Candida albicans* hyphae in GE medium with addition of 5% C3, and Fig. 2F is a schematic diagram showing the growth of *Candida albicans* hyphae in GE medium with addition of 5% C4.
Fig. 3A is a schematic diagram showing a blank control for HBD2 immunohistochemical detection.
Fig. 3B is a schematic diagram showing a positive control for HBD2 immunohistochemical detection.
FIG. 3C is a schematic diagram showing the S1 sample group for HBD2 immunohistochemical detection.
Fig. 3D is a schematic diagram showing a blank control for HBD3 immunohistochemical detection.
Fig. 3E is a schematic diagram showing a positive control for HBD3 immunohistochemical detection.
Fig. 3F is a schematic diagram showing the S1 sample group for HBD3 immunohistochemical detection.

### STRAIN DEPOSIT INFORMATION

The bacterial strain *Lactobacillus rhamnosus* 11-7 used in the present application was deposited in the China Center for Type Culture Collection (CCTCC) on February 22, 2021, and the accession number is CCTCC NO: M 2021185. Address: Wuhan University, Wuhan city, China; postcode: 430072; Tel: (027)-68754052.

### DETAILED DESCRIPTION OF THE APPLICATION

The present application will be described in detail below with reference to the embodiments in conjunction with the drawings, wherein the same numbers in all the drawings indicate the same features. Although specific Examples of the application are shown in the drawings, it should be understood that the application may be embodied in various forms and is not limited to the Examples set forth herein. On the contrary, these Examples are provided for thoroughly understanding of the present application and fully conveying the scope of the present application to those skilled in the art.

It should be noted that, certain terms are used in the specification and claims to refer to specific components. Those skilled in the art should understand that they may use different terms to refer to the same component. The specification and claims do not use differences in nouns as a way of distinguishing components, but use differences in functions of components as a criterion for distinguishing. "Comprises" or "includes" mentioned throughout the specification and claims are open-ended terms, so it should be interpreted as "including but not limited to". The following descriptions in the specification are preferred embodiments for implementing the present application. However, these descriptions are for the purpose of the general principles of the specification, and are not intended to limit the scope of the present application. The protection scope of the present application should be defined by the appended claims.

The application provides a bacterial strain *Lactobacillus rhamnosus* 11-7, which is preserved in China Center for Type Culture Collection with accession number CCTCC NO: M 2021185.

The bacterial strain is isolated and purified from newborn breast milk.

The present application provides use of the above bacterial strain in the field of fermentation, preferably in the field of producing ferment lysates by fermentation.

In the present application, the above bacterial strain is used for fermentation. *Lactobacillus rhamnosus* is widely used as an excellent probiotic in the fields of food, medicine and cosmetics as a safe bacterial variety, and breast milk is used as the first protective barrier for newborns. The activity of probiotics in breast milk is self-evident, and all the active substances in the final product may be well retained by separating and screening the probiotics colonized in breast milk and then preparing ferment lysates through the process of the present application; accordingly, the activity and efficacy of this strain is far superior to traditional probiotics in terms of source, strain type, and preparation process.

The application provides a ferment lysate, which comprises proteins, polysaccharides and amino acids, wherein based on the mass percentage in the ferment lysate, the content of the proteins is 0.2-1%, preferably 0.6-0.9%; the content of the polysaccharides is 0.3-0.8%, preferably 0.4-0.6%; and the content of the amino acids is 0.2-1%, preferably 0.4-0.5%.

For example, based on the mass percentage in the ferment lysate, the content of the proteins is 0.2%, 0.3%, 0.4%, 0.5%, 0.6%, 0.7%, 0.8%, 0.9%, 1.0%, etc.

The content of the polysaccharides is 0.3%, 0.4%, 0.5%, 0.6%, 0.7%, 0.8% etc.

The content of the amino acids is 0.2%, 0.3%, 0.4%, 0.5%, 0.6%, 0.7%, 0.8%, 0.9%, 1.0% and so on.

In one embodiment, the ferment lysate is obtained by performing fermentation and enzymolysis by using *Lactobacillus rhamnosus* strain 11-7 with accession number CCTCC NO: M 2021185, preferably, the ferment lysate is prepared by a method comprising the steps of:
inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells;
performing enzymolysis of the fermentation bacterial cells to obtain a ferment lysate.

The fermentation medium contains carbon source, nitrogen source and inorganic salt well known by those skilled in the art. The carbon source can be, for example, glucose, lactose, sucrose, maltose or mannitol, preferably glucose. The addition amount of the carbon source can be 1-5% (w/v), preferably 1-4% (w/v).

The nitrogen source can be, for example, beef extract, peptone, yeast powder, bean cake powder, etc., preferably peptone, and the addition amount of the nitrogen source can be 0.5-2wt%, preferably 1-2wt%.

The inorganic salt can be, for example, magnesium sulfate, dipotassium hydrogen phosphate, sodium chloride, manganese chloride, etc., preferably magnesium sulfate and dipotassium hydrogen phosphate; the addition amount of magnesium sulfate can be 0.1-1wt%, preferably 0.5- 0.8wt%; and the addition amount of dipotassium hydrogen phosphate can be 0.1-0.5wt%, preferably 0.1-0.2wt%.

The inoculum amount is 0.1-0.5%.

In one embodiment, the enzymes used in the enzymolysis are lysozyme, neutral protease and snailase, preferably the specific enzyme activity of the lysozyme is 2000-20000IU/mg, and the specific enzyme activity of the neutral protease is 50000-100000IU/g.

The specific enzyme activity refers to the enzyme activity per milligram of the product.

The lysozyme, also known as muramidase or N-acetylmuramide glycanohydrlase, is an alkaline enzyme capable of hydrolyzing mucopolysaccharides in bacteria. Lysozyme mainly breaks the β-1,4 glycosidic bond between N-acetylmuramic acid and N-acetylglucosamine in the cell wall, decomposes the insoluble mucopolysaccharide in the cell wall into soluble glycopeptide, and makes the cell wall broken, thereby releasing the contents and lysing the bacteria.

The neutral protease is obtained by fermentation and extraction of *Bacillus subtilis,* belongs to an endonuclease, and can be used for various protein hydrolysis treatments. Under a certain temperature and pH value, it can hydrolyze macromolecular proteins into products such as amino acids. It can be widely used in the enzymolysis and refining process of the protein products of animals, plants, and microbes. The neutral protease can act on soluble polypeptides in cell walls, and the combination with lysozyme can be used to completely degrade them.

The snailase is a mixed enzyme prepared from snail bursa and digestive tract, which contains more than 20 kinds of enzymes such as cellulase, pectinase, amylase and protease. Snailase is a valuable enzyme. It can be used for breaking the cell wall of yeast, so it is widely used in the research of cell biology and genetic engineering; it can be used as a feed additive to improve the digestibility of feed; and it can also be used for juice clarification, orange decapsulation, and jam making, etc.

For example, the specific enzyme activity of the lysozyme is 2000IU/mg, 3000IU/mg, 4000IU/mg, 5000IU/mg, 6000IU/mg, 7000IU/mg, 8000IU/mg, 9000IU/mg, 10000IU/mg, 11000IU/mg , 12000IU/mg, 13000IU/mg, 14000IU/mg, 15000IU/mg, 16000IU/mg, 17000IU/mg, 18000IU/mg, 19000IU/mg, or 20000IU/mg, etc.

The specific enzyme activity of the neutral protease is 5000IU/mg, 6000IU/mg, 7000IU/mg, 8000IU/mg, 9000IU/mg, or 10000ILT/mg, etc.

For snailase, its wall-breaking rate is 95%. The wall-breaking refers to a fact that in a sorbitol solution with pH5.8-7.2, the cell walls may be lysed after keeping 10-20 mg of enzyme per gram of cells at 37°C for 1 hour. In one embodiment, the time period of enzymolysis is 1-2 hours, and the temperature of enzymolysis is 35-40°C.

For example, the time period of enzymolysis is 1h, 1.5h, or 2h, etc., and the temperature of enzymolysis is 35°C, 36°C, 37°C, 38°C, 39°C, or 40°C, etc.

In one embodiment, for 1 g of the fermentation bacterial cells, the addition amount of lysozyme is 0.01-0.1 mg, the addition amount of snailase is 1-10 mg, and the addition amount of neutral protease is 0.01-0.1mg.

For example, for 1 g of fermentation bacterial cells, the addition amount of lysozyme can be 0.01 mg, 0.02 mg, 0.03 mg, 0.04 mg, 0.05 mg, 0.06 mg, 0.07 mg, 0.08 mg, 0.09 mg, or 0.1 mg, etc.

The addition amount of snailase can be 1 mg, 2 mg, 3 mg, 4 mg, 5 mg, 6 mg, 7 mg, 8 mg, 9 mg, or 10 mg, etc.

The addition amount of neutral protease can be 0.01mg, 0.02mg, 0.03mg, 0.04mg, 0.05mg, 0.06mg, 0.07mg, 0.08mg, 0.09mg, or 0.1mg, etc.

The above-mentioned enzymes are used in the application to perform enzymolysis of the fermentation bacterial cells, a mild way of crushing is used to inactivate and crush the fermentation bacterial cells, after crushing a variety of active ingredients as small molecules that are easily absorbed, such as amino acids, polysaccharides, polypeptides, etc. can be formed, so that the materials are transformed from cell morphology that cannot be absorbed by the human skin to a small molecule state that can be easily absorbed.

The present application does not make any restrictions on the method of adding the above enzymes. For example, the three enzymes can be added after mixing, or they can be added one by one. The present application does not make any restrictions on the order of adding one by one, and it can be selected according to the well-known methods in the art.

In one embodiment, the supernatant obtained after centrifugation of the first fermentation broth is filtered through a filter membrane to obtain a second fermentation broth.

In one embodiment, after enzymolysis, before obtaining the ferment lysate, the method for preparing a ferment lysate further comprises the following steps:
centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.

In the present application, sodium chloride is used for suspending, thereby resulting in a hypertonic environment to degrade the cell wall after being treated with enzymes, and the cell will be broken to release the remaining intracellular substances due to hypertonicity, thereby forming bacterial cell debris; sodium chloride is mainly used to cause a hypertonic environment.

In the present application, the supernatant obtained from centrifugation of the first fermentation broth (i.e. a second fermentation broth) is used for suspending. Since the second fermentation broth contains extracellular active substances in the fermentation process, the ferment lysate contains extracellular active substances after the suspending.

The application provides a method for preparing a ferment lysate, the method comprises the step of:
preparing a ferment lysate by using *Lactobacillus rhamnosus* 11-7 strain with accession number CCTCC NO: M 2021185.

In one embodiment, wherein the ferment lysate is obtained by performing fermentation and enzymolysis by using the bacterial strain.

In one embodiment, the method comprises the steps of:
inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells;

Preferably, the inoculation amount is 0.1-0.5%.

Preferably, the fermentation is carried out at a temperature of 35-38°C, and the fermentation time is 40-70 hours. Within this time range, the first fermentation broth is obtained after the saccharide is exhausted.

The present application is not limited to the preparation of bacterial strains in logarithmic phase, which can be prepared using common methods in the art, for example, inoculating the bacterial strain into MRS liquid medium, and culturing at 37°C for 15-20h to obtain the bacterial strains in logarithmic phase.

In one embodiment, the enzymes used in the enzymolysis are lysozyme, neutral protease and snailase, preferably the specific enzyme activity of the lysozyme is 2000-20000IU/mg, and the specific enzyme activity of the neutral protease is 50000-100000IU/g; preferably, the time period of the enzymolysis is 1-2h, and the temperature of the enzymolysis is 35-40°C;

Preferably, for 1 g of the fermentation bacterial cells, the addition amount of lysozyme is 0.01-0.1 mg, the addition amount of snailase is 1-10 mg, and the addition amount of neutral protease is 0.01-0.1mg.

In one embodiment, the supernatant obtained after centrifuging the first fermentation broth is filtered through a filter membrane to obtain a second fermentation broth.

In the present application, by treating the bacterial cells and the fermentation supernatant separately, the addition amount of the enzymolysis solution can be reduced, the risk of skin allergies can be reduced, and the active ingredients existing in the extracellular supernatant can also be retained. The whole process not only maintains the types and activities of functional substances in the whole fermentation, but also converts them into forms that are easy to absorb.

The filter membrane is a 0.22 µm filter membrane.

In one embodiment, after enzymolysis, before obtaining the ferment lysate, the method further comprises the following steps:
centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.

The precipitate of bacterial cells refers to a precipitate obtained by centrifuging after enzymolysis, then removing the supernatant after centrifugation.

The bacterial cell debris refers to a precipitate obtained by suspending the precipitate of bacterial cells in a sodium chloride solution, and then centrifuging and removing the supernatant.

The sodium chloride solution is a sodium chloride solution that has been filtered and sterilized, and the concentration of the sodium chloride is 3-5 wt%.

In one embodiment, the mass-volume percentage of the cell debris in the second fermentation broth is 1-10%, preferably 5-8%.

For example, the mass-volume percentage of the cell debris in the second fermentation broth can be 1%, 2%, 3%, 4%, 5%, 6%, 7%, 8%, 9%, or 10%, etc.

In one embodiment, the ferment lysate comprises proteins, polysaccharides and amino acids, wherein based on the mass percentage in the ferment lysate, the content of the proteins is 0.2-1%, preferably 0.6-0.9%; the content of the polysaccharides is 0.3-0.8%, preferably 0.4-0.6%; and the content of the amino acids is 0.2-1%, preferably 0.4-0.5%.

The ferment lysate of the present application can inhibit the reproduction and growth of pathogenic bacteria, promote some probiotics, and have a strong inhibitory effect on the pathogenic hyphae of *Candida albicans.* Additionally, the bacterial strain itself can not only generate antimicrobial peptides during fermentation, but also up-regulate the expression level of antimicrobial peptides in skin cells at the genetic level and the protein level, thereby improving skin immunity, and as a cosmetic raw material it can significantly regulate the skin micro-ecological environment.

In the present application, the bacterial cells are inactivated and crushed through a mild crushing way by performing enzymolysis with enzyme preparation in combination with hypertonic solution. After crushing the bacterial cells, a variety of active ingredients as small molecules that are easily absorbed, such as amino acids, polysaccharides, polypeptides, etc. can be formed, so that the materials are transformed from cell morphology that cannot be absorbed by the human skin to a small molecule state that can be easily absorbed. Moreover, by treating the bacterial cells and the fermentation supernatant separately, it is possible to reduce the amount of enzymolysis solution to be added, reduce the risk of skin allergies, and also preserve the extracellular active ingredients present in the supernatant. The whole process not only maintains the types and activities of functional substances in the whole fermentation, but also converts them into forms that are easy to absorb. The production cycle is short, and the process is controllable and easy to scale up. The product is stable with high content of active substances which is easy to absorb, thereby having good efficacy. The product is also safe and healthy, thereby conforming to the characteristics of cosmetic raw materials.

The present application provides use of the above ferment lysate or the ferment lysate prepared by the above preparation method in the field of cosmetics.

Preferably, the ferment lysate is used to regulate skin microecology.

### EXAMPLES

The present application generally and/or specifically describes the materials and test methods used in the experiments. In the following examples, if there is no other special explanation, % means wt%, i.e., weight (mass) percentage. The reagents or instruments used in the application, whose manufacturers are not indicated, are all commercially available conventional reagent products.

### Example 1: The Acquisition of Bacterial Strain

Newborn breast milk was collected, diluting the breast milk 10 times by mixing with non-sterile purified water, taking 100 µL to spread on solid MRS plate, incubating in 37°C incubator for 48 hours. A single colony grown on the plate was diluted to spread on solid MRS plate again for separating and screening, incubating in 37°C incubator for 48 hours. The morphology and physiological and biochemical experiments of single colonies are as follows.

### 1.1 Morphological observation

The bacterial strain was spread on a glass slide, performing Gram staining to observe the morphology of bacterial cells. The bacterial colonies were spread on solid MRS plate by applicator to incubate at 37°C for 24 hours overnight, the state of single colony on the plate was observed.

### 1.2 Physiological and biochemical experiments

A bacterial strain was obtained after separation and purification, then the following physiological and biochemical experiments were conducted: fermentation experiment for gas production, catalase reaction, starch hydrolysis reaction, casein hydrolysis reaction, phenylalanine deaminase reaction, citrate reaction, indole reaction, gelatin liquefaction reaction, nitrate reduction reaction, and litmus milk reaction.

**Table 1: The morphological observation of bacterial colony under the microscope**

| Gram's staining | Bacterial morphology | Spore morphology | Flagella |
|---|---|---|---|
| Positive | Short rod shape | None | None |

**Table 2: The morphological observation of bacterial colony on the plate**

| Colony shape | Marginal morphology | Surface | Color |
|---|---|---|---|
| Regular circle | Neat | Wet, smooth, and raised | White |

**Table 3: Physiological and biochemical experiments**

| Physiological and biochemical items | Experimental results |
|---|---|
| Fermentation reaction for gas production | - |
| Catalase reaction | - |
| Starch hydrolysis reaction | - |
| Casein hydrolysis reaction | - |
| Phenylalanine deaminase reaction | - |
| Citrate reaction | - |
| Indole reaction | - |
| Gelatin liquefaction reaction | - |
| Nitrate reduction reaction | - |
| Litmus milk reaction | + |

From the above identification results, this bacterial strain can be preliminarily judged to belong to the genus Lactobacillus.

### 1.3 Identification of the bacterial strain

1) The main materials and reagents are shown in Table 4.

**Table 4:**

| Reagent Name | Supplier | Catalog |
|---|---|---|
| Ezup Column Bacterial Genomic DNA Extraction Kit | Sangon Biotech | B518259 |
| SanPrep Column DNA Gel Recovery Kit | Sangon Biotech | B518131 |
| Taq Plus DNA Polymerase | BBI | B600090 |
| Agarose B | BBI | A600014 |
| 4S Red Plus Nucleic Acid Stain (10,000X Aqueous Solution) | BBI | A606695 |
| GeneRuler DNA Ladder Mix | Thermo Scientific | B300721 |

2) The main instruments are shown in Table 5.

**Table 5:**

| Name | Manufacturer | Product type |
|---|---|---|
| Clean bench | Jiangsu Sujie Clean Equipment Factory | SW-CJ-1D |
| PCR reaction amplifier | BBI | PCR-96 |
| High-speed microcentrifuge, 100-14800 rpm | Sangon Biotech | G508009 |
| Electrophoresis | Beijing Liuyi Equipment Factory | DYY-6C |
| Gel imaging system | Shanghai Furi Science & Technology Co., Ltd. | FR980 |
| Micro-spectrophotometer | Merinton Instrument, Inc. | SMA4000 |
| Sequencer | ABI, Foster, CA, USA | 3730XL |

3. Experimental steps
3.1 The primer sequences are respectively shown in SEQ ID NO:1 and SEQ ID NO:2, wherein the nucleotide sequences are as follows:
27F: AGAGTTTGATCMTGGCTCAG (SEQ ID NO: 1)
1492R: GGTTACCTTGTTACGACTT (SEQ ID NO: 2)

3.3 DNA extraction
Detailed operation steps see the instructions of Ezup Column Bacterial Genomic DNA Extraction Kit (B518259).
3.4 PCR amplification
3.4.1 The PCR reaction system is shown in Table 6.

**Table 6:**

| Reaction components | Volume (µl) |
|---|---|
| 10 X PCR Buffer | |
| dNTP (each 10 mM) | |
| Taq Plus DNA Polymerase (5 U/µl) | |
| 50mM MgSO₄ | 12.5 in total |
| Primer F (10 µM) | 1 |
| Primer R (10 µM) | 1 |
| Template (DNA) | 1 |
| ddH₂O | 9.5 |
| Total | 25 |

3.4.2 The conditions for PCR reaction are shown in Table 7.

**Table 7:**

| Temperature (°C) | Time | Cycle |
|---|---|---|
| 95 | 5 min | |
| 94 | 30 s | 30 cycles |
| 57 | 30 s | |
| 72 | 90 s | |
| 72 | 10 min | |

3.4.3 PCR electrophoresis

1.5% agarose gel, 1x TAE, 150V, 100mA, 20min electrophoresis, the observation results are shown in Fig. 1.

The bacterial strain was sequenced and identified by Sangon Biotech (Shanghai) Co., Ltd. The sequencing result of 16S rDNA is shown in SEQ ID NO: 3, after comparison by Blast the bacterial strain was proved *Lactobacillus rhamnosus* 11-7. Particularly, the nucleotide sequence is as follows:

TTTGATCATGGCTCAGGATGAATACTGGCGGCGTGCCTAATACATGCAAGTCGAACGAGTTCTGATTATTGAAAGGTGCT
TGCATCTTGATTTAATTTTGAACGAGTGGCGGACGGGTGAGTAACACGTGGGTAACCTGCCCTTAAGTGGGGGATAACA
TTTGGAAACAGATGCTAATACCGCATAAATCCAAGAACCGCATGGTTCTTGGCTGAAAGATGGCGTAAGCTATCGCTTTT
GGATGGACCCGCGGCGTATTAGCTAGTTGGTGAGGTAACGGCTCACCAAGGCAATGATACGTAGCCGAACTGAGAGGTT
GATCGGCCACATTGGGACTGAGACACGGCCCAAACTCCTACGGGAGGCAGCAGTAGGGAATCTTCCACAATGGACGCA
AGTCTGATGGAGCAACGCCGCGTGAGTGAAGAAGGCTTTCGGGTCGTAAAACTCTGTTGTTGGAGAAGAATGGTCGGC
AGAGTAACTGTTGTCGGCGTGACGGTATCCAACCAGAAAGCCACGGCTAACTACGTGCCAGCAGCCGCGGTAATACGT
AGGTGGCAAGCGTTATCCGGATTTATTGGGCGTAAAGCGAGCGCAGGCGGTTTTTTAAGTCTGATGTGAAAGCCCTCGG
CTTAACCGAGGAAGTGCATCGGAAACTGGGAAACTTGAGTGCAGAAGAGGACAGTGGAACTCCATGTGTAGCGGTGA

AATGCGTAGATATATGGAAGAACACCAGTGGCGAAGGCGGCTGTCTGGTCTGTAACTGACGCTGAGGCTCGAAAGCAT
GGGTAGCGAACAGGATTAGATACCCTGGTAGTCCATGCCGTAAACGATGAATGCTAGGTGTTGGAGGGTTTCCGCCCTT
CAGTGCCGCAGCTAACGCATTAAGCATTCCGCCTGGGGAGTACGACCGCAAGGTTGAAACTCAAAGGAATTGACGGGG
GCCCGCACAAGCGGTGGAGCATGTGGTTTAATTCGAAGCAACGCGAAGAACCTTACCAGGTCTTGACATCTTTTGATCA
CCTGAGAGATCAGGTTTCCCCTTCGGGGGCAAAATGACAGGTGGTGCATGGTTGTCGTCAGCTCGTGTCGTGAGATGTT
GGGTTAAGTCCCGCAACGAGCGCAACCCTTATGACTAGTTGCCAGCATTTAGTTGGGCACTCTAGTAAGACTGCCGGTG
ACAAACCGGAGGAAGGTGGGGATGACGTCAAATCATCATGCCCCTTATGACCTGGGCTACACACGTGCTACAATGGATG
GTACAACGAGTTGCGAGACCGCGAGGTCAAGCTAATCTCTTAAAGCCATTCTCAGTTCGGACTGTAGGCTGCAACTCGC
CTACACGAAGTCGGAATCGCTAGTAATCGCGGATCAGCACGCCGCGGTGAATACGTTCCCGGGCCTTGTACACACCGCC
CGTCACACCATGAGAGTTTGTAACACCCGAAGCCGGTGGCGTAACCCTTTTAGGGAGCGAGCCGTCTAAGGTGGGACA
AATGATTAGGGTGAAGTCGTAA

### Example 2-1

*Lactobacillus rhamnosus* strain was picked from the test tube to inoculate in 500mL of sterile seed culture solution containing MRS, standing still to cultivate at 37°C for 18 hours until the logarithmic growth phase, then inoculating again to 5L fermentation medium with 0.5% inoculum amount. The fermentation medium contains 2 wt% of glucose, 1.5 wt% of peptone, 0.1 wt% of dipotassium hydrogen phosphate and 0.5 wt% of magnesium sulfate, cultivating at 37°C for 50h without residual saccharide; after the fermentation is completed, the first fermentation broth is obtained to denote as fermentation broth 1.

### Example 2-2

*Lactobacillus rhamnosus* strain was picked from the test tube to inoculate in 100mL of sterile seed culture solution containing MRS, standing still to cultivate at 37°C for 20 hours until the logarithmic growth phase, then inoculating again to 3L fermentation medium with 0.3% inoculum amount. The fermentation medium contains 4 wt% of glucose, 2 wt% of peptone, 0.2 wt% of dipotassium hydrogen phosphate and 0.8 wt% of magnesium sulfate, cultivating at 37°C for 65h without residual saccharide; after the fermentation is completed, the first fermentation broth is obtained to denote as fermentation broth 2.

### Example 2-3

*Lactobacillus rhamnosus* strain was picked from the test tube to inoculate in 100mL of sterile seed culture solution containing MRS, standing still to cultivate at 37°C for 15 hours until the logarithmic growth phase, then inoculating again to 3L fermentation medium with 0.5% inoculum amount. The fermentation medium contains 4 wt% of glucose, 2 wt% of peptone, 0.2 wt% of dipotassium hydrogen phosphate and 0.8 wt% of magnesium sulfate, cultivating at 37°C for 65h without residual saccharide; after the fermentation is completed, the first fermentation broth is obtained to denote as fermentation broth 3.

### Example 2-4

*Lactobacillus rhamnosus* strain was picked from the test tube to inoculate in 100mL of sterile seed culture solution containing MRS, standing still to cultivate at 37°C for 20 hours until the logarithmic growth phase, then inoculating again to 3L fermentation medium with 0.5% inoculum amount. The fermentation medium contains 2 wt% of glucose, 1 wt% of peptone, 0.1 wt% of dipotassium hydrogen phosphate and 0.5 wt% of magnesium sulfate, cultivating at 37°C for 65h without residual saccharide; after the fermentation is completed, the first fermentation broth is obtained to denote as fermentation broth 4.

### Example 2-5

*Lactobacillus rhamnosus* strain was picked from the test tube to inoculate in 100mL of sterile seed culture solution containing MRS, standing still to cultivate at 37°C for 20 hours until the logarithmic growth phase, then inoculating again to 3L fermentation medium with 0.2% inoculum amount. The fermentation medium contains 1 wt% of glucose, 0.5 wt% of peptone, 0.1 wt% of dipotassium hydrogen phosphate and 0.1 wt% of magnesium sulfate, cultivating at 37°C for 65h without residual saccharide; after the fermentation is completed, the first fermentation broth is obtained to denote as fermentation broth 5.

### Example 2-6

*Lactobacillus rhamnosus* strain was picked from the test tube to inoculate in 100mL of sterile seed culture solution containing MRS, standing still to cultivate at 37°C for 20 hours until the logarithmic growth phase, then inoculating again to 3L fermentation medium with 0.5% inoculum amount. The fermentation medium contains 5 wt% of glucose, 0.8 wt% of peptone, 0.5 wt% of dipotassium hydrogen phosphate and 1 wt% of magnesium sulfate, cultivating at 37°C for 65h without residual saccharide; after the fermentation is completed, the first fermentation broth is obtained to denote as fermentation broth 6.

### Example 2-7

*Lactobacillus rhamnosus* strain was picked from the test tube to inoculate in 100mL of sterile seed culture solution containing MRS, standing still to cultivate at 37°C for 20 hours until the logarithmic growth phase, then inoculating again to 3L fermentation medium with 0.5% inoculum amount. The fermentation medium contains 5 wt% of glucose, 0.8 wt% of peptone, 0.5 wt% of dipotassium hydrogen phosphate and 1 wt% of magnesium sulfate, cultivating at 37°C for 65h without residual saccharide; after the fermentation is completed, the first fermentation broth is obtained to denote as fermentation broth 7.

### Example 3-1

(1) Fermentation broth 7 (200mL) was taken to collect the fermentation bacterial cells by centrifuging at 8000rpm and obtain 12.2g fermentation bacterial cells, wherein the supernatant is filtered through a 0.22 micron filter membrane to obtain a second fermentation broth.
(2) The fermentation bacterial cells (12.2g) was suspended in 1.2L purified water, adding 0.122mg lysozyme (specific enzyme activity is 5000IU/mg), 0.122mg neutral protease (specific enzyme activity is 60000IU/mg), and 73.2mg snailase to mix well, conducting enzymolysis at 37°C for 1 hour, then centrifuging and discarding the supernatant to obtain a bacterial precipitate; suspending the bacterial precipitate in 100ml of 4% sodium chloride solution sterilized by filtration, standing still for 1 hour after suspension, then centrifuging and discarding the supernatant to obtain cell debris; the cell debris (9.4g) was suspended in 160mL of the second fermentation broth to obtain a ferment lysate (S1), and S1 was subjected to component analysis, wherein the protein was determined by method of Coomassie brilliant blue as follows:
   Acidic staining solution was prepared as reagent: 0.1g of Coomassie brilliant blue G250 was taken to add 50ml of ethanol to dissolve, adding 100ml of phosphoric acid, and adding water to dilute to 1000ml and mix well; filtering to obtain a filtrate (i.e., the reagent). This reagent should be stored in a brown bottle, and if there is precipitation, it must be filtered before use.

Preparation of reference substance solution: unless otherwise specified, a reference substance of serum albumin (bovine) or national standard substance for protein content determination was taken to add water to dissolve and make a solution containing 1mg protein per 1ml.

Preparation of test solution: it was prepared according to the method specified under each item (the protein concentration should be basically the same as that of the reference substance solution).

Determination method: 0.0ml, 0.01ml, 0.02ml, 0.04ml, 0.06ml, 0.08ml, and 0.1ml of the reference substance solution (the amount of the reference substance solution can be adjusted appropriately within the measurement range of this method) was precisely measured to put into test tubes with stopper respectively; for each of the test tubes, water was added into it to a volume of 0.1ml, then adding 5.0ml of acidic staining solution to mix well immediately, and measuring the absorbance immediately at a wavelength of 595nm according to the ultraviolet-visible spectrophotometry method (general rule 0401); at the same time, tube No. 0 was used as blank control.

The linear regression equation was calculated with the concentrations of the reference substance solutions and the corresponding absorbance. In addition, an appropriate amount of the test solution was accurately measured to test by the same method, the protein concentration of the test solution was calculated by the linear regression equation, multiplying it by the dilution factor to obtain a protein content of 0.9%.

Determination of amino acid content: the amino acid content was determined by using amino acid automatic analyzer (LA8080), and the amino acid content is 0.5%.

Determination of polysaccharide: the polysaccharide content was determined by phenol sulfuric acid method.
(1) Instruments: visible-ultraviolet spectrophotometer, analytical balance (precision 0.0001g), vortex mixer
(2) Reagents:
   2.1 Standard solution: 100mg of glucose (analytical pure) with dry constant weight was accurately weighed to put into a volumetric flask, adding water to make up to 250mL, then shaking up and accurately drawing 10mL of the solution to add water to make up to 100mL.
   2.2 Preparation of 80% phenol solution: 80 mL of double-distilled phenol was accurately pipetted to add distilled water to make up to 100 mL and obtain 80% phenol solution, storing in a brown bottle away from light.
   2.3 Preparation of 6% phenol solution: 80% phenol solution was diluted to 6% with water, and it was made immediately before use.
   2.4 Concentrated sulfuric acid (guaranteed reagent)
(3) Detection:
   3.1 Making a standard curve: 0.0, 0.4, 0.8, 1.2, 1.6, and 2.0 mL of glucose standard solutions were drawn into test tubes with stopper, and for each of the test tubes, water was added to make up to 2.0 mL, adding 1.0mL of 6% phenol solution respectively to mix well, then quickly adding 5.0mL of concentrated sulfuric acid (the concentrated sulfuric acid was added by hanging in the air, and cannot adhere to the wall) to shake and mix well immediately. After reacting at room temperature for 20 minutes, the absorbance was measured at 490nm, and the No. 0 tube was used as a blank control; a standard curve was obtained by using the concentration of polysaccharides as vertical coordinate, and the absorbance as horizontal coordinate.
   3.2 Preparing samples: 0.2ml of each of the samples S1-S4 in Examples 5-8 was taken respectively to put into a 50mL volumetric flask, adding an appropriate amount of water; after complete dissolution, water was added to the mark to set the volume as a stock solution, then shaking to mix well. 5mL of the stock solution was taken before use, then putting it into a 50mL volumetric flask, adding water to the mark, then diluting 10 times again in the same way. 2mL was taken to put into a test tube with stopper, starting from the above mentioned (Section 3.1) operation step of "adding 1.0mL of 6% phenol solution", performing in the same way. The polysaccharide concentration of the sample to be tested was obtained by the standard curve, the polysaccharide content was calculated according to the dilution factor to obtain a polysaccharide content of 0.6% .

### Example 3-2

(1) Fermentation broth 7 (200mL) was taken to collect the fermentation bacterial cells by centrifuging at 8000rpm and obtain 15.8g fermentation bacterial cells, wherein the supernatant is filtered through a 0.22 micron filter membrane to obtain a second fermentation broth.
(2) The fermentation bacterial cells (15.8g) was suspended in 316mL purified water, adding 0.79mg lysozyme (specific enzyme activity is 15000IU/mg), 0.79mg neutral protease (specific enzyme activity is 80000IU/mg), and 79mg snailase to mix well, conducting enzymolysis at 38°C for 1 hour, then centrifuging and discarding the supernatant to obtain a bacterial precipitate; suspending the bacterial precipitate in 120ml of 4% sodium chloride solution sterilized by filtration, standing still for 1 hour after suspension, then centrifuging and discarding the supernatant to obtain cell debris; the cell debris (13.9g) was suspended in 200mL of the second fermentation broth to obtain a ferment lysate (S2), then analyzing according to the method described in Example 3-1 to obtain a protein content of 0.6%, an amino acid content of 0.4%, and a polysaccharide content of 0.4%.

### Example 3-3

(1) Fermentation broth 7 (200mL) was taken to collect the fermentation bacterial cells by centrifuging at 8000rpm and obtain 13.7g fermentation bacterial cells, wherein the supernatant is filtered through a 0.22 micron filter membrane to obtain a second fermentation broth.
(2) The fermentation bacterial cells (13.7g) was suspended in 137mL purified water, adding 1.37mg lysozyme (specific enzyme activity is 14000IU/mg), 1.37mg neutral protease (specific enzyme activity is 80000IU/mg), and 137mg snailase to mix well, conducting enzymolysis at 38°C for 1 hour, then centrifuging and discarding the supernatant to obtain a bacterial precipitate; suspending the bacterial precipitate in 150ml of 3% sodium chloride solution sterilized by filtration, standing still for 1 hour after suspension, then centrifuging and discarding the supernatant to obtain cell debris; the cell debris (11.5g) was suspended in 150mL of the second fermentation broth to obtain a ferment lysate (S3), then analyzing according to the method described in Example 3-1 to obtain a protein content of 0.7%, an amino acid content of 0.5%, and a polysaccharide content of 0.6%.

### Example 3-4

(1) Fermentation broth 7 (200mL) was taken to collect the fermentation bacterial cells by centrifuging at 8000rpm and obtain 16.1g fermentation bacterial cells, wherein the supernatant is filtered through a 0.22 micron filter membrane to obtain a second fermentation broth.
(2) The fermentation bacterial cells (16.1g) was suspended in 330mL purified water, adding 0.161mg lysozyme (specific enzyme activity is 8000IU/mg), 1.61mg neutral protease (specific enzyme activity is 90000IU/mg), and 80.5mg snailase to mix well, conducting enzymolysis at 38°C for 1 hour, then centrifuging and discarding the supernatant to obtain a bacterial precipitate; suspending the bacterial precipitate in 200ml of 4% sodium chloride solution sterilized by filtration, standing still for 1 hour after suspension, then centrifuging and discarding the supernatant to obtain cell debris; the cell debris (13.0g) was suspended in 130mL of the second fermentation broth to obtain a ferment lysate (S4), then analyzing according to the method described in Example 3-1 to obtain a protein content of 0.3%, an amino acid content of 0.3%, and a polysaccharide content of 0.8%.

### Example 3-5

(1) Fermentation broth 7 (200mL) was taken to collect the fermentation bacterial cells by centrifuging at 8000rpm and obtain 11.4g fermentation bacterial cells, wherein the supernatant is filtered through a 0.22 micron filter membrane to obtain a second fermentation broth.
(2) The fermentation bacterial cells (11.4g) was suspended in 380mL purified water, adding 0.114mg lysozyme (specific enzyme activity is 5000IU/mg), 0.114mg neutral protease (specific enzyme activity is 60000IU/mg), and 114mg snailase to mix well, conducting enzymolysis at 37°C for 1 hour, then centrifuging and discarding the supernatant to obtain a bacterial precipitate; suspending the bacterial precipitate in 100ml of 4% sodium chloride solution sterilized by filtration, standing still for 1 hour after suspension, then centrifuging and discarding the supernatant to obtain cell debris; the cell debris (8.5g) was suspended in 150mL of the second fermentation broth to obtain a ferment lysate (S5), then analyzing according to the method described in Example 3-1 to obtain a protein content of 0.9%, an amino acid content of 0.3%, and a polysaccharide content of 0.9%.

### Example 3-6

(1) Fermentation broth 7 (200mL) was taken to collect the fermentation bacterial cells by centrifuging at 8000rpm and obtain 13.1g fermentation bacterial cells, wherein the supernatant is filtered through a 0.22 micron filter membrane to obtain a second fermentation broth.
(2) The fermentation bacterial cells (13.1g) was suspended in 260mL purified water, adding 0.655mg lysozyme (specific enzyme activity is 5000IU/mg), 0.131mg neutral protease (specific enzyme activity is 60000IU/mg), and 78.6mg snailase to mix well, conducting enzymolysis at 37°C for 1 hour, then centrifuging and discarding the supernatant to obtain a bacterial precipitate; suspending the bacterial precipitate in 100ml of 4% sodium chloride solution sterilized by filtration, standing still for 1 hour after suspension, then centrifuging and discarding the supernatant to obtain cell debris; the cell debris (10.2g) was suspended in 170mL of the second fermentation broth to obtain a ferment lysate (S6), then analyzing according to the method described in Example 3-1 to obtain a protein content of 0.3%, an amino acid content of 0.8%, and a polysaccharide content of 0.7%.

### Example 3-7

(1) Fermentation broth 7 (100mL) was taken to collect the fermentation bacterial cells by centrifuging at 8000rpm and obtain 5.4g fermentation bacterial cells, wherein the supernatant is filtered through a 0.22 micron filter membrane to obtain a second fermentation broth.
(2) The fermentation bacterial cells (5.4g) was suspended in 108mL purified water, adding 0.54mg lysozyme (specific enzyme activity is 5000IU/mg), 0.054mg neutral protease (specific enzyme activity is 60000IU/mg), and 32.4mg snailase to mix well, conducting enzymolysis at 37°C for 1 hour, then centrifuging and discarding the supernatant to obtain a bacterial precipitate; suspending the bacterial precipitate in 100ml of 4% sodium chloride solution sterilized by filtration, standing still for 1 hour after suspension, then centrifuging and discarding the supernatant to obtain cell debris; the cell debris (3.6g) was suspended in 60mL of the second fermentation broth to obtain a ferment lysate (S7), then analyzing according to the method described in Example 3-1 to obtain a protein content of 0.2%, an amino acid content of 0.3%, and a polysaccharide content of 0.3%.

### Example 3-8

(1) Fermentation broth 7 (300mL) was taken to collect the fermentation bacterial cells by centrifuging at 8000rpm and obtain 21.0g fermentation bacterial cells, wherein the supernatant is filtered through a 0.22 micron filter membrane to obtain a second fermentation broth.
(2) The fermentation bacterial cells (21.0g) was suspended in 420mL purified water, adding 0.21mg lysozyme (specific enzyme activity is 5000IU/mg), 0.21mg neutral protease (specific enzyme activity is 60000IU/mg), and 105mg snailase to mix well, conducting enzymolysis at 37°C for 1 hour, then centrifuging and discarding the supernatant to obtain a bacterial precipitate; suspending the bacterial precipitate in 100ml of 4% sodium chloride solution sterilized by filtration, standing still for 1 hour after suspension, then centrifuging and discarding the supernatant to obtain cell debris; the cell debris (15.5g) was suspended in 250mL of the second fermentation broth to obtain a ferment lysate (S8), then analyzing according to the method described in Example 3-1 to obtain a protein content of 0.5%, an amino acid content of 0.2%, and a polysaccharide content of 0.7%.

### Example 3-9

(1) Fermentation broth 7 (500mL) was taken to collect the fermentation bacterial cells by centrifuging at 8000rpm and obtain 32.4g fermentation bacterial cells, wherein the supernatant is filtered through a 0.22 micron filter membrane to obtain a second fermentation broth.
(2) The fermentation bacterial cells (32.4g) was suspended in 650mL purified water, adding 3.24mg lysozyme (specific enzyme activity is 10000ILT/mg), 1.62mg neutral protease (specific enzyme activity is 90000IU/mg), and 324mg snailase to mix well, conducting enzymolysis at 38°C for 1 hour, then centrifuging and discarding the supernatant to obtain a bacterial precipitate; suspending the bacterial precipitate in 600ml of 5% sodium chloride solution sterilized by filtration, standing still for 1 hour after suspension, then centrifuging and discarding the supernatant to obtain cell debris; the cell debris (19.5g) was suspended in 1950mL of the second fermentation broth to obtain a ferment lysate (S9), then analyzing according to the method described in Example 3-1 to obtain a protein content of 0.4%, an amino acid content of 0.6%, and a polysaccharide content of 0.3%.

**Table 8: The process conditions in the Examples**

| | Addition amount of lysozyme per gram of bacterial cells (mg) | Addition amount of neutral protease per gram of bacterial cells (mg) | Addition amount of snailase per gram of bacterial cells (mg) | Mass-volume percentage of the cell debris in the second fermentation broth |
|---|---|---|---|---|
| Example 3-1 | 0.01 | 0.01 | 6 | 6% |
| Example 3-2 | 0.05 | 0.05 | 5 | 7% |
| Example 3-3 | 0.1 | 0.1 | 10 | 8% |
| Example 3-4 | 0.01 | 0.1 | 5 | 10% |
| Example 3-5 | 0.01 | 0.01 | 10 | 6% |
| Example 3-6 | 0.05 | 0.01 | 6 | 6% |
| Example 3-7 | 0.1 | 0.01 | 6 | 6% |
| Example 3-8 | 0.01 | 0.01 | 5 | 6% |
| Example 3-9 | 0.1 | 0.05 | 10 | 1% |

### Comparative Example 1

Fermentation broth 7 (200mL) was taken to put into 60°C water bath and heat for 1h to obtain a comparative sample of *Lactobacillus* lysate to denote as C1, then analyzing according to the method described in Example 3-1 to obtain a protein content of 0.1%, an amino acid content of 0.15%, and a polysaccharide content of 0.2%.

### Comparative Example 2

A commercially available lysate product of *Lactobacillus bifidus* was selected as a comparative sample to denote as C2 (commercially available), which has a protein content of 0.1%, an amino acid content of 0.07%, and a polysaccharide content of 0.2%.

### Comparative Example 3

Fermentation broth 7 (200mL) was taken to collect the bacterial cells by centrifuging at 8000rpm, and the supernatant is filtered through a 0.22 micron filter membrane for later use. The bacterial cells were suspended in 260mL of purified water, adding 2.1mg lysozyme, 2.1mg neutral protease, and 63mg snailase to mix well. The wet weight of the above bacterial cells collected from fermentation broth 7 is 10.5g, and the bacterial cells was suspended in 100ml enzymatic hydrolysate, performing enzymolysis at 37°C for 1.5 hours, then centrifuging to discard the supernatant. After enzymolysis of the fermentation broth, a total of 9.0g cell debris was obtained, suspending in 4% sodium chloride solution sterilized by filtration with a final volume of 100ml, standing still for 1 hour, then centrifuging to discard the supernatant; the resulting cell precipitate was suspended in 130ml supernatant from the sterilized fermentation broth 1 to denote as C3, then analyzing according to the method described in Example 3-1 to obtain a protein content of 0.2%, an amino acid content of 0.1%, and a polysaccharide content of 0.1%.

### Comparative Example 4

The process conditions of Comparative Example 4 are identical to those of Example 3-7 except that in Comparative Example 4, ferment lysate (C4) was obtained by using commercially available *Lactobacillus rhamnosus;* then analyzing according to the method described in Example 3-1 to obtain a protein content of 0.1%, an amino acid content of 0.3%, and a polysaccharide content of 0.2%.

### Test Example 1

MRS solid plates were prepared, the samples of lysate from each of the Examples and Comparative Examples were taken respectively, then using spreader to apply 100 µL of the primary lysate, applying 100 µL of the 10 times diluted primary lysate, and 100 µL of the 100 times primary lysate suspension on the plates, and placing them in incubators to culture at 37°C for 48 hours. The single bacterial colonies on the plates were counted, and the results are shown in Table 9 below.

**Table 9: Number of single bacterial colonies on the plates**

| | Number of bacterial colonies on the plates of the primary lysates | | Number of bacterial colonies on the plates of the 10 times diluted primary lysates | | Number of bacterial colonies on the plates of the 100 times diluted primary lysate | |
|---|---|---|---|---|---|---|
| Example 3-1 | | 0 | | 0 | | 0 |
| Example 3-2 | | 0 | | 0 | | 0 |
| Example 3-3 | | 0 | | 0 | | 0 |
| Example 3-4 | | 1 | | 0 | | 0 |
| Example 3-5 | | 1 | | 0 | | 0 |
| Example 3-6 | | 0 | | 1 | | 0 |
| Example 3-7 | | 1 | | 0 | | 0 |
| Example 3-8 | | 1 | | 0 | | 0 |
| Example 3-9 | | 0 | | 1 | | 0 |
| Comparative Example 1 | | 56 | | 16 | | 0 |
| Comparative Example 2 | | 34 | | 4 | | 0 |
| Comparative Example 3 | | 21 | | 3 | | 0 |
| Comparative Example 4 | | 16 | | 3 | | 0 |

It can be seen from the above experimental results that the bacterial cells cannot be crushed by the method of the Comparative Examples and the growth of them were not affected, while the crushing effect of the Examples were relatively ideal, indicating that a better crushing effect may be obtained by using a specific process and a specific bacterial strain.

### Test Example 2

The samples of lysates from each of the Examples and Comparative Examples were taken to determine the content of amino acids by using an automatic amino acid analyzer, and the results are shown in Table 10.

**Table 10: The Results of the content of Amino Acids**

| Name of Amino acid | Content of Amino acids (mg/100mL) | | | | | | | | | | | | |
|---|---|---|---|---|---|---|---|---|---|---|---|---|---|
| | Example 3-1 | Example 3-2 | Example 3-3 | Example 3-4 | Example 3-5 | Example 3-6 | Example 3-7 | Example 3-8 | Example 3-9 | Compara tive Example 1 | Compara tive Example 2 | Compara tive Example 3 | Compara tive Example 4 |
| Asp | 21.7 | 18.9 | 23.1 | 20.1 | 15.2 | 40.9 | 12.6 | 10.9 | 22.2 | 7.3 | 1.2 | 0.7 | 5.4 |
| Thr | 17.0 | 25.5 | 22.5 | 19.9 | 17.7 | 39.7 | 20.5 | 9.9 | 34.6 | 0.0 | 0.6 | 0.0 | 3.2 |
| Ser | 21.2 | 23.5 | 28.5 | 21.4 | 15.3 | 42.6 | 30.1 | 11.4 | 21.3 | 12.1 | 5.1 | 1.2 | 20.6 |
| Gin | 63.6 | 50.2 | 75.6 | 25.9 | 60.1 | 88.7 | 10.1 | 31.3 | 75.5 | 8.6 | 3.3 | 8.6 | 80.5 |
| Gly | 9.6 | 6.6 | 10.2 | 11.3 | 7.7 | 31.6 | 7 | 5.6 | 7.6 | 7.3 | 0.0 | 7.3 | 10.1 |
| Cys | 27.0 | 17.4 | 28.1 | 19.6 | 14.6 | 50.3 | 25.5 | 7 | 55.3 | 4.7 | 4.5 | 4.7 | 14.4 |
| Val | 5.8 | 3.1 | 6.2 | 5.9 | 4.3 | 11.7 | 4.9 | 2.5 | 3.3 | 6.0 | 3.0 | 6.0 | 10.5 |
| Met | 27.4 | 25.5 | 27.5 | 25.5 | 20.5 | 45.8 | 22.1 | 12.5 | 21.4 | 1.7 | 5.9 | 1.7 | 4.3 |
| Ile | 14.0 | 18.9 | 19.5 | 22.4 | 14.5 | 25.2 | 30.6 | 10.9 | 13.9 | 6.4 | 4.0 | 6.4 | 5.1 |
| Leu | 20.4 | 19.0 | 22.4 | 17.5 | 14.4 | 25.5 | 10.1 | 9.8 | 15.8 | 6.4 | 3.7 | 6.3 | 6.6 |
| Tyr | 61.8 | 50.5 | 74.5 | 23.5 | 50.5 | 100.5 | 32.4 | 34.6 | 110.4 | 5.5 | 10.9 | 5.5 | 50.5 |
| Phe | 21.0 | 22.2 | 25.8 | 18.4 | 16.3 | 49.9 | 20.6 | 9.5 | 20.6 | 5.1 | 11.2 | 5.2 | 11.7 |
| Lys | 50.8 | 50.4 | 55.4 | 32.5 | 28.8 | 60.3 | 40.7 | 25.1 | 60.4 | 8.3 | 1.6 | 8.9 | 26.7 |
| His | 56.7 | 50.8 | 61.4 | 30.4 | 30.4 | 80.5 | 26.4 | 5.2 | 34.5 | 49.0 | 14.5 | 49.1 | 20.3 |
| Arg | 11.5 | 10.4 | 15.4 | 14.3 | 10 | 31.9 | 23.1 | 7.4 | 31.5 | 16.7 | 0.6 | 16.7 | 10.4 |
| Pro | 37.7 | 34.7 | 44.1 | 30.5 | 21.1 | 45.1 | 20.1 | 16.4 | 60.6 | 4.1 | 1.2 | 4.5 | 20.1 |
| Trp | 15.1 | 11.4 | 20.5 | 15.5 | 10.2 | 30.2 | 14.6 | 9.9 | 15.4 | 0.8 | 0.0 | 0.0 | 1.5 |
| Total | 493.3 | 439.0 | 560.7 | 354.6 | 351.6 | 800.4 | 351.4 | 219.9 | 604.3 | 150.0 | 71.3 | 133 | 301.9 |

### Test Example 3

The samples of lysates from each of the Examples and Comparative Examples were taken to respectively dilute 10 times, Oxford Cup Method was used for antimicrobial test, and three kinds of bacteria i.e., *Staphylococcus aureus, Pseudomonas aeruginosa,* and *Escherichia coli* were selected as the indicator bacteria of pathogenic bacteria. The inhibitory effects of the samples on the pathogenic bacteria were compared, and the results are shown in Table 11 below.

**Table 11: The diameters of the inhibition zones of different samples**

| | Diameter of the inhibition zone (mm) | | |
|---|---|---|---|
| | *Staphylococcus aureus* | *Pseudomonas aeruginosa* | *Escherichia coli* |
| Example 3-1 | 47 | 53 | 38 |
| Example 3-2 | 45 | 51 | 39 |
| Example 3-3 | 49 | 55 | 38 |
| Example 3-4 | 40 | 41 | 30 |
| Example 3-5 | 36 | 42 | 31 |
| Example 3-6 | 42 | 40 | 29 |
| Example 3-7 | 40 | 38 | 26 |
| Example 3-8 | 42 | 39 | 30 |
| Example 3-9 | 41 | 34 | 33 |
| Comparative Example 1 | 20 | 30 | 18 |
| Comparative Example 2 | 10 | 21 | -- |
| Comparative Example 3 | 15 | 10 | -- |
| Comparative Example 4 | 25 | 33 | 10 |

It can be seen from Table 11 that, the ferment lysates prepared in the Examples can inhibit the growth of pathogenic bacteria such as *Staphylococcus aureus, Pseudomonas aeruginosa,* and *Escherichia coli.*

### Test Example 4

The pathogenicity of *Candida albicans* hyphae is related to morphological transformation. *Candida albicans* can grow in three forms, i.e., yeast form, pseudohyphae form and hyphae form. Pseudohyphal state can produce germ tube-specific antigen, which can significantly enhance its adhesion and prevent phagocytic killing effect of neutrophils. After *Candida albicans* is swallowed, it can still generate germ tubes and grow hyphae in the phagocytes, thereby penetrating and destroying the host cell membrane. Experiments have shown that, *Candida albicans* growing in the form of hyphae are more pathogenic. The normal resident *Candida albicans* is in the yeast form, which only exists on the surface of keratinocytes and does not penetrate into them. When infected by *Candida albicans,* germ tubes are firstly formed, and adhere to the surface of host cells with the help of structures such as adhesin in the outermost layer of the cell wall, and then the germ tubes gradually transform into budding hyphae or hyphae form, and penetrate into the cells to grow, when presenting in the form of hyphae it shows strong pathogenicity.

GE medium (commercially available) was used to induce the hyphae growth of *Candida albicans* (purchased from American Type Culture Collection with accession number ATCC10231), and 5% concentration of the samples of lysates from each of the Example 3-1 and Comparative Examples 1 to 4 were respectively added to the *Candida albicans* medium to culture for 6 hours. The inhibition effect on the germination of *Candida albicans* hyphae were compared, and the growth results are shown in Figs. 2A-2F, respectively.

It can be seen from these figures that, the sample S1 can well inhibit the germination of the pathogenic hyphae of *Candida albicans.*

### Test Example 5

Ferment lysates of *Lactobacillus* have immunoregulatory and chemotactic effects. When the skin is stimulated by the outside world, skin cell-related antimicrobial peptides such as defensins and other factors will be significantly up-regulated. With the increase of age, the skin tends to age; and the expression of antimicrobial peptides in the skin is significantly reduced. The samples of ferment lysates from each of the Examples and Comparative Examples were taken to apply to keratinocytes with an addition amount of 0.4%, and the expression of antimicrobial peptide genes HBD2 and HBD3 related to skin immunity in keratinocytes was detected, then evaluating the regulatory effects of the three samples on skin immune-related genes.

The above keratinocytes were treated with RNAiso Plus and then collected, conducting RNA extraction, reverse transcription and fluorescent quantitative PCR operation according to the instructions of the kit, and analyzing the results by the relative quantitative method. The detection steps are as follows:
1. Pouring off the culture solution, and washing once with 1*PBS.
2. Adding 1-2mL of RNAiso Plus to 10 cm² cultured cells, and shaking to ensure that the RNAiso Plus lysis solution is evenly distributed on the cell surface.
3. Transferring the lysis solution containing cells to a centrifugal tube, and repeatedly blowing and sucking with a pipette until there is no obvious precipitation in the lysis solution.
4. Standing at room temperature for 5 minutes, and then separating RNA from protein.
5. Adding chloroform to the above solution, closing the centrifuge cap tightly to mix until the solution is milky white, then standing still for 5 minutes.
6. Centrifuging at 12,000g at 4°C for 15 minutes, then carefully taking the centrifugal tube from the centrifuge; at this time, the colorless supernatant contains RNA.
7. Pipetting the supernatant into a new centrifugal tube.
8. Adding isopropanol having 0.5-1 times the volume of RNAiso Plus to the supernatant, inverting the centrifugal tube up and down to mix well, and standing still at room temperature for 10 minutes.
9. Centrifuging at 12,000g at 4°C for 10min, carefully discarding the supernatant to leave a small amount of isopropanol, adding 75% ethanol having the same volume of RNAiso Plus, gently inverting the centrifugal tube upside down to wash the tube wall, then centrifuging at 15,000g at 4°C for 5min, and carefully discarding the supernatant; do not touch the precipitate.
10. Opening the centrifugal tube to dry the precipitate at room temperature for a few minutes, after drying the precipitate, adding an appropriate amount of RNase-free water to dissolve the precipitate.
11. After the RNA was extracted, the cDNA template was synthesized according to the reaction system indicated in the reverse transcription kit, and then the fluorescent quantitative PCR was carried out according to the reaction system indicated in the real-time PCR kit (Shanghai GenePharma Co., Ltd.). The results are shown as follows.

The change trend of the test results is shown in Table 12.

**Table 12: Expression levels of HBD2 and HBD3**

| Name of gene | HBD2 | HBD3 |
|---|---|---|
| BC | 1.025 | 1.025 |
| Example 3-1 | 2.241 | 2.241 |
| Example 3-2 | 2.161 | 2.443 |
| Example 3-3 | 2.282 | 2.519 |
| Example 3-4 | 1.875 | 1.507 |
| Example 3-5 | 1.686 | 1.654 |
| Example 3-6 | 1.990 | 1.342 |
| Example 3-7 | 1.643 | 1.872 |
| Example 3-8 | 1.701 | 1.545 |
| Example 3-9 | 1.797 | 1.411 |
| Comparative Example 1 | 1.201 | 1.132 |
| Comparative Example 2 | 0.995 | 1.010 |
| Comparative Example 3 | 1.121 | 1.242 |
| Comparative Example 4 | 1.337 | 1.122 |

It can be seen from Table 12 that the expression levels of HBD2 and HBD3 in the ferment lysate obtained by using the bacterial strain of the present application to ferment and performing enzymolysis by the method of the present application are relatively high, thereby it is indicated that ferment lysate of the present application has immunoregulatory and chemotactic effects.

### Test Example 6

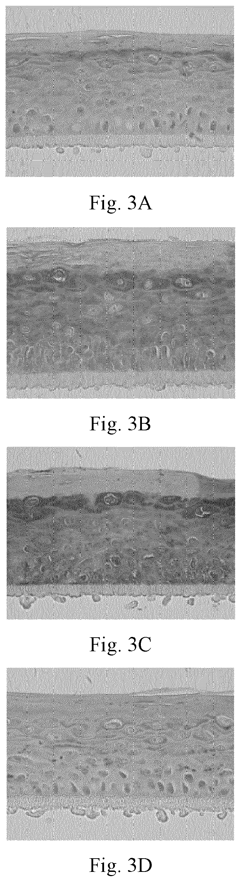

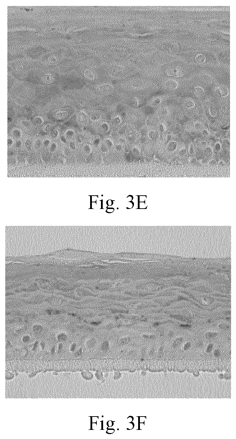

In order to further verify the activity of the ferment lysate of *Lactobacillus,* its effect on the expression of skin antimicrobial peptides was evaluated on a 3D skin model. In the S1 sample group, the ferment lysate of *Lactobacillus* obtained in Example 3-1 was added to the 3D skin model with a concentration of 39 µL/cm². In the positive control group, 50mJ/cm² UVB was used to continuously irradiate the 3D skin model for two days, and it was used for the detection of HBD2 and HBD3 proteins. The blank control was a 3D skin model without treatment. The steps are as follows:
1. Transferring the 3D skin model (EpiKutis^{®}, produced by Guangdong Biocell Biotech Co., Ltd.) to a 6-well plate (adding 0.9mL model culture solution in advance), and marking the test group number on the 6-well plate.
2. Sample group: 25 µL of the prepared working solution for S1 sample group was taken to add to the surface of the model;
   Blank control: 25 µL of EpiGrowth (Guangdong Biocell Biotech) culture solution was taken to add to the surface of the model;
   Positive control: 25 µL of EpiGrowth (Guangdong Biocell Biotech) culture solution was taken to add to the surface of the model, then continuously irradiating the skin model with 50mJ/cm² UVB for 48 hours;
   Gently shaking the model to distribute the sample evenly on the surface of the model, and then placing it in a CO₂ incubator (37°C, 5% CO₂) to incubate for 24h.
3. After the incubation, washing the remaining test substance on the surface of the model with a washing bottle filled with sterile PBS solution, and gently wiping off the residual liquid inside and outside the model with a sterile cotton swab.
4. Ring-cutting the model off: the model used for immunohistochemical detection was fixed, paraffin-embedded, and sliced.
5. Baking the slices to dewax: the paraffin slices were placed in a 70°C roaster to bake for 4 hours.
6. Dewaxing and hydration: the slices was soaked in xylene for 10 minutes, soaking again in newly replaced xylene for another 10 minutes, soaking in absolute ethanol for 5 minutes, soaking in 95% ethanol for 5 minutes, and then soaking in 75% ethanol for 5 minutes; washing with PBS buffer 3 times, Smin each time.
7. Antigen retrieval: the paraffin slices were put into an antigen retrieval solution of 0.01M sodium citrate, repairing with high pressure, and taking out the slices after cooling; washing with PBS buffer 3 times, Smin each time.
8. Blocking peroxidase (horseradish catalase DAB chromogenic kit (Shanghai Sangong)): 1 drop of 3% H₂O₂ was added to each slice, then incubating at room temperature for 30min to block the activity of endogenous peroxidase; washing with PBS buffer 3 times, Smin each time.
9. Serum blocking: serum homologous to the secondary antibody was added dropwise to block for 60 minutes at 37°C without washing.
10. Primary antibody incubation: working solution of primary antibody was added dropwise to incubate overnight at 4°C; washing with PBS buffer 3 times, Smin each time; wherein mouse or goat recombinant Anti-HBD2 (Abcam)/Anti-HBD3 (Abcam) were used as the primary antibody respectively, and PBS buffer containing 3% BSA (bovine serum albumin) was used to dilute it at a ratio of 1:100-1:500 to prepare a working solution of primary antibody.
11. Secondary antibody incubation: a working solution of secondary antibody was added dropwise to incubate at room temperature for 1 h, washing with PBS buffer 3 times, Smin each time; wherein goat anti-mouse or rabbit anti-goat antibody was selected as the secondary antibody, and PBS buffer was used to dilute it at a ratio of 1:20-1:50 to prepare a working solution of the secondary antibody.
12. Incubation in ABC complex solution: ABC complex solution (VECTASTAIN^{®}, ABC-Peroxidase Kits) was added dropwise to incubate at room temperature for 30 min; washing with PBS buffer 3 times, Smin each time.
13. DAB staining: 1 drop of freshly prepared DAB solution was added to each slice (specific parts will be stained brown) to observe under the microscope for 5-30s.
14. Counterstaining: counterstaining with hematoxylin for 30s.
15. Dehydration: the slices were dehydrated by graded alcohol (75%, 95%, 100%, and 100%) for 5 minutes each, then soaking in xylene for 10 minutes, soaking again in newly replaced xylene for another 10 minutes, drying, and sealing with neutral resin; drying in the air, then observe the slices. The results are shown in Fig. 3A-3F, wherein Fig. 3A is the schematic diagram of the HBD2 immunohistochemical detection of the blank control, Fig. 3B is the schematic diagram of the HBD2 immunohistochemical detection of the positive control, Fig. 3C is the schematic diagram of the HBD2 immunohistochemical detection of the S1 sample group, Fig. 3D is a schematic diagram of the HBD3 immunohistochemical detection of the blank control, Fig. 3E is a schematic diagram of the HBD3 immunohistochemical detection of the positive control, and Fig. 3F is a schematic diagram of the HBD3 immunohistochemical detection of the S1 sample group.

ImageJ was used to compare the integrated optical density (IOD) values of HBD2 and HBD3 by the grayscale comparison method, performing relative quantitative analysis on the graphs. The results are shown in Tables 13-14 below, respectively.

**Table 13: Analysis results of the HBD2 integrated optical density (IOD) values**

| Name of the sample | Relative average IOD |
|---|---|
| Blank control | 1.00 |
| Positive control | 1.38 |
| S1 sample group | 1.39 |

**Table 14: Analysis results of the HBD3 integrated optical density (IOD) values**

| Name of the sample | Relative average IOD |
|---|---|
| Blank control | 1.00 |
| Positive control | 1.22 |
| S1 sample group | 1.02 |

It can be seen from Tables 13-14 that compared with the blank group, the HBD2 protein of the ferment lysate of *Lactobacillus* in the sample group can be significantly increased by 39% in the skin 3D model, which is higher than that in the positive control, while the effect of HBD3 protein is not obvious .

In summary, The ferment lysate obtained by using the *Lactobacillus rhamnosus* of the present application for fermentation and performing enzymolysis can strongly inhibit the reproduction and growth of pathogenic bacteria, has a promotion effect on some of probiotics, especially having strong inhibition effect on pathogenic hyphae of *Candida albicans*; and during fermentation process, it can not only produce antimicrobial peptides, but also increase the expression of antimicrobial peptides in skin cells at the gene level and protein level, thereby improving skin immunity; as a cosmetic raw material it can significantly regulate the skin micro-ecological environment.

The above Examples are only preferred examples of the present application, and are not intended to limit the other forms of present application. Any skilled person who is familiar with this technical field may use the technical content disclosed above to change or modify into equivalent examples. However, any simple modifications, equivalent changes and modifications made to the above Examples based the technical essence of the present application without departing from the content of the technical solution of the present application shall fall into to the protection scope of the technical solution of the present application.

## Claims

1. A *Lactobacillus rhamnosus* 11-7 strain, which is preserved in the China Center for Type Culture Collection (CCTCC) with accession number CCTCC NO: M 2021185.

2. Use of *Lactobacillus rhamnosus* 11-7 strain with accession number CCTCC NO: M 2021185 in the field of fermentation, preferably in the field of producing ferment lysates by fermentation.

3. A ferment lysate, which comprises proteins, polysaccharides and amino acids, wherein based on the mass percentage in the ferment lysate, the content of the proteins is 0.2-1%, preferably 0.6-0.9%; the content of the polysaccharides is 0.3-0.8%, preferably 0.4-0.6%; and the content of the amino acids is 0.2-1%, preferably 0.4-0.5%.

4. The ferment lysate according to claim 3, wherein the ferment lysate is obtained by performing fermentation and enzymolysis by using *Lactobacillus rhamnosus* strain with accession number CCTCC NO: M 2021185,
preferably, the ferment lysate is prepared by a method comprising the steps of:
inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells; preferably, the fermentation medium contains carbon source, nitrogen source and inorganic salt; and preferably, the addition amount of the carbon source is 1-5% (w/v), preferably 1-4% (w/v), the addition amount of the nitrogen source is 0.5-2wt%, and the addition amount of the inorganic salt is 0.1-1wt%; and
performing enzymolysis of the fermentation bacterial cells to obtain a ferment lysate.

5. The ferment lysate according to claim 4, wherein the enzymes used in the enzymolysis are lysozyme, neutral protease and snailase; preferably, the specific enzyme activity of the lysozyme is 2000-20000IU/mg, and the specific enzyme activity of the neutral protease is 50000-100000 IU/g.

6. The ferment lysate according to claim 4 or 5, wherein the time period of the enzymolysis is 1-2h, and the temperature of the enzymolysis is 35-40°C.

7. The ferment lysate according to any one of claims 5-6, wherein for 1 g of the fermentation bacterial cells, the addition amount of lysozyme is 0.01-0.1 mg, the addition amount of snailase is 1-10 mg, and the addition amount of neutral protease is 0.01-0.1mg.

8. The ferment lysate according to any one of claims 4-7, wherein the supernatant obtained after centrifugation is filtered through a filter membrane to obtain a second fermentation broth.

9. The ferment lysate according to claim 8, wherein after enzymolysis, before obtaining the ferment lysate, the method for preparing a ferment lysate further comprises the following steps:
centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.

10. The ferment lysate according to claim 9, wherein the concentration of the sodium chloride is 3-5wt%.

11. The ferment lysate according to claim 9 or 10, wherein the mass-volume percentage of the cell debris in the second fermentation broth is 1-10%, preferably 5-8%.

12. A method for preparing a ferment lysate, comprising the step of:
preparing a ferment lysate by using *Lactobacillus rhamnosus* 11-7 strain with accession number CCTCC NO: M 2021185.

13. The method according to claim 12, wherein the ferment lysate is obtained by performing fermentation and enzymolysis by using the bacterial strain.

14. The method according to claim 13, wherein the method comprises the steps of:
inoculating the bacterial strain in logarithmic phase into a fermentation medium for fermentation until there is no residual saccharide to obtain a first fermentation broth, centrifuging the first fermentation broth and removing the supernatant to obtain fermentation bacterial cells; preferably, the fermentation medium contains carbon source, nitrogen source and inorganic salt; and preferably, the addition amount of the carbon source is 1-5% (w/v), preferably 1-4% (w/v), the addition amount of the nitrogen source is 0.5-2wt%, and the addition amount of the inorganic salt is 0.1-1wt%; and
performing enzymolysis of the fermentation bacterial cells to obtain a ferment lysate.

15. The method according to claim 13 or 14, wherein the enzymes used in the enzymolysis are lysozyme, neutral protease and snailase; preferably, the specific enzyme activity of the lysozyme is 2000-20000IU/mg, and the specific enzyme activity of the neutral protease is 50000-100000 IU/g.

16. The method according to claim 14 or 15, wherein the time period of the enzymolysis is 1-2h, and the temperature of the enzymolysis is 35-40°C.

17. The method according to claim 15 or 16, wherein for 1 g of the fermentation bacterial cells, the addition amount of lysozyme is 0.01-0.1 mg, the addition amount of snailase is 1-10 mg, and the addition amount of neutral protease is 0.01-0.1mg.

18. The method according to any one of claims 14-17, wherein the supernatant obtained after centrifugation is filtered through a filter membrane to obtain a second fermentation broth.

19. The method according to claim 18, wherein after enzymolysis, before obtaining the ferment lysate, the method further comprises the following steps:
centrifuging after the enzymolysis to obtain precipitate of bacterial cells, and suspending the precipitate in sodium chloride solution, then centrifuging to obtain debris of bacterial cells, and suspending the debris in the second fermentation broth to obtain the ferment lysate.

20. The method according to claim 19, wherein the concentration of the sodium chloride is 3-5 wt%.

21. The method according to claim 19 or 20, wherein the mass-volume percentage of the cell debris in the second fermentation broth is 1-10%, preferably 5-8%.

22. Use of the ferment lysate according to any one of claims 3-11 or the ferment lysate prepared by the method according to any one of claims 12-21 in the field of cosmetics.

23. The use according to claim 22, wherein the ferment lysate is used for regulating skin microecology.
